# EUROPEAN PATENT SPECIFICATION

(11) **EP 2 828 380 B1**
(45) Date of publication and mention of the grant of the patent: **21.09.2016**
(21) Application number: 14703899.6
(22) Date of filing: 23.01.2014
(51) Int. Cl.: C12N 5/079, C12N 5/0797

(54) **METHODS AND MEDIA FOR DIFFERENTIATING EYE CELLS**
VERFAHREN UND MEDIEN ZUR UNTERSCHEIDUNG VON AUGENZELLEN
PROCÉDÉS ET MILIEUX POUR DIFFÉRENCIER DES CELLULES OCULAIRES

(30) Priority: 03.04.2013 FI 20135318
(43) Date of publication of application: 28.01.2015
(73) Proprietor: Tampereen Yliopisto, 33014 Tampereen Yliopisto (FI)
(72) Inventor: MIKHAILOVA, Alexandra, 33014 Tampereen yliopisto (FI); ILMARINEN, Tanja, 33014 Tampereen yliopisto (FI); SKOTTMAN, Heli, 33014 Tampereen yliopisto (FI)
(74) Representative: Kolster Oy Ab
(86) International application number: PCT/FI2014/050053
(87) International publication number: WO 2014/162040

(56) References cited:
- EP-A1- 1 835 023
- EP-A2- 2 410 044
- WO-A1-2008/129554
- WO-A1-2009/051671
- WO-A1-2010/144696
- WO-A1-2012/073238
- WO-A1-2012/103012
- WO-A2-2011/043591
- WO-A2-2011/063005
- WO-A2-2011/159726
- US-A1- 2007 020 247
- HANNA VAAJASAARI ET AL: "Toward the defined and xeno-free differentiation of functional human pluripotent stem cell-derived retinal pigment epithelial cells.", MOLECULAR VISION, vol. 17, 1 January 2011 (2011-01-01), pages 558-575, XP055108414, ISSN: 1090-0535 cited in the application
- ASTRID SUBRIZI ET AL: "Generation of hESC-derived retinal pigment epithelium on biopolymer coated polyimide membranes", BIOMATERIALS, vol. 33, no. 32, 1 November 2012 (2012-11-01), pages 8047-8054, XP055108413, ISSN: 0142-9612, DOI: 10.1016/j.biomaterials.2012.07.033
- OSAKADA F ET AL: "Toward the generation of rod and cone photoreceptors from mouse, monkey and human embryonic stem cells", NATURE BIOTECHNOLOGY, NATURE PUBLISHING GROUP, NEW YORK, NY, US, vol. 26, no. 2, 1 February 2008 (2008-02-01), pages 215-224, XP008117904, ISSN: 1087-0156, DOI: 10.1038/NBT1384 [retrieved on 2008-02-26] cited in the application
- OSAKADA FUMITAKA ET AL: "Stepwise differentiation of pluripotent stem cells into retinal cells", NATURE PROTOCOLS, NATURE PUBLISHING GROUP, GB, vol. 4, no. 6, 1 January 2009 (2009-01-01) , pages 811-824, XP001525355, ISSN: 1750-2799
- MARIA IDELSON ET AL: "Directed Differentiation of Human Embryonic Stem Cells into Functional Retinal Pigment Epithelium Cells", CELL STEM CELL, vol. 5, no. 4, 1 October 2009 (2009-10-01) , pages 396-408, XP055046648, ISSN: 1934-5909, DOI: 10.1016/j.stem.2009.07.002
- IKEDA H ET AL: "Generation of Rx+/Pax6+ neural retinal precursors from embryonic stem cells", PROCEEDINGS OF THE NATIONAL ACADEMY OF SCIENCES, NATIONAL ACADEMY OF SCIENCES, US, vol. 102, no. 32, 1 August 2005 (2005-08-01), pages 11331-11336, XP002991777, ISSN: 0027-8424, DOI: 10.1073/PNAS.0500010102
- IAN YAT-HIN WONG ET AL: "Promises of stem cell therapy for retinal degenerative diseases", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 249, no. 10, 25 August 2011 (2011-08-25), pages 1439-1448, XP019955765, ISSN: 1435-702X, DOI: 10.1007/S00417-011-1764-Z
- ALEXANDRA MIKHAILOVA ET AL: "Small-Molecule Induction Promotes Corneal Epithelial Cell Differentiation from Human Induced Pluripotent Stem Cells", STEM CELL REPORTS, vol. 2, no. 2, 1 February 2014 (2014-02-01), pages 219-231, XP055108416, ISSN: 2213-6711, DOI: 10.1016/j.stemcr.2013.12.014
- ANNI SORKIO ET AL: "Structure and Barrier Properties of Human Embryonic Stem Cell-Derived Retinal Pigment Epithelial Cells Are Affected by Extracellular Matrix Protein Coating", TISSUE ENGINEERING PART A, 20 January 2014 (2014-01-20), XP055108412, ISSN: 1937-3341, DOI: 10.1089/ten.tea.2013.0049

## Description

### FIELD OF THE INVENTION

The technical field concerned is treatment and study of the eye, more specifically corneal diseases and disorders. The present description is related to differentiation of stem cells into eye precursor cells and further into differentiated eye cells, such as corneal epithelial cells. Accordingly, here is provided means and methods contributing to fast and effective induction, maturation and differentiation.

### BACKGROUND OF THE INVENTION

The cornea is located at the front surface of the eye and is multilayered, transparent and avascular in structure. The main functions of the cornea are to protect the eyeball and its contents, while allowing accurate focusing of light to produce a sharp image on the retina. The cornea consists of three cellular layers, namely epithelium, stroma and endothelium, separated by two acellular layers - Bowman's layer, and Descemet membrane. As the outermost layer, corneal epithelium is exposed to the external environment and thereby needs to be rapidly regenerating and stratified. Similarly to the epidermis, lens and conjunctival epithelium, corneal epithelium originates from the surface ectoderm. However, detailed developmental mechanisms and signaling routes remain unknown.

In a prior art publication, Hayashi et al, 2012 reported corneal cell differentiation on PA6 cells (feeder cells of mouse origin) taking 12-16 weeks. The differentiation efficiency measured with expression of CK12 protein was less than 15%. Better efficiency and xeno-free conditions are still desired.

Hanson et al, 2012 published differentiation on Bowman's membrane for 25 days. The efficiency was not, however, reported. The Bowman's membrane is known being unstable, which casts uncertainty to the procedure.

Yoshida et al, 2011 managed to produce precursor cells by differentiation of mouse induced pluripotent stem cells through cultivation on PA6 cells (feeder cells of mouse origin). Differentiation took about 60 days. There still is need for xeno-free differentiation conditions.

Ahmad et al, 2007 published culturing of hESC on collagen IV. They used medium conditioned by the limbal fibroblasts which resulted in the loss of pluripotency and differentiation into epithelial like cells. They reported differentiation efficiency of 50% on day 5 and 10% on day 21 as measured by expression of proteins CK3/12. Use of a medium which requires donated limbal cells can be considered problematic. Moreover, there is significant biological variation between batches of limbal cells.

Retinal pigment epithelium (RPE) is an epithelial cell monolayer located between the neural retina and choriocapillaris. RPE provides essential support for the long-term preservation of retinal integrity and visual functions by absorbing stray light, regenerating visual pigment, supplying nutrients, secreting growth factors, and phagocytosing the shed photoreceptor outer segments (POS). Dysfunctional RPE causes impairment and death of the photoreceptor cells, leading to deterioration or total loss of vision. These mechanisms play an important role in the pathogenesis of retinal diseases like age-related macular degeneration (AMD), which is the leading cause of blindness in the developed world.

Human pluripotent stem cells may serve as an unlimited source of RPE cells for transplantation. Several groups have reported successful RPE differentiation originating from human embryonic stem cells (hESCs) and human induced pluripotent stem cells (hiPSCs). However, current differentiation methods for RPE cells mainly rely either on spontaneous differentiation processes favoring neuroectodermal lineage, which is characteristic of hESCs, or on a complex mixture of several different growth factors, inhibitors or other functional ingredients.

Osakada et al., 2008 and 2009 succeeded in enhancing RPE differentiation efficiency with a prolonged culture period and cell culture supplements. Studied supplements include: nicotinamide (NIC), Activin A, transforming growth factor beta (TGFβ), Wnt signaling inhibitor casein kinase I inhibitor (CKI)-7, dickkopf-related protein-1 (Dkk-1), Lefty-Δ, fibroblast growth factor antagonist Y-27632, and nodal signaling inhibitor SB431542. Regardless of the improvement of the differentiation efficacy, reaching a sufficient amount of maturated cells with RPE characteristics still demanded long-term differentiation processes.

WO 2013/184809 discloses a relatively fast method for the derivation of RPE cells from pluripotent cells. However, the method is very complex with at least four different stages and four different culture media, each characterized by a unique combination of active supplement.

Xeno-products and undefined factors used in many differentiation processes pose further challenges, because animal-derived components may carry factors such as sialic acid or Neu5Gc, causing unwanted immunogenicity of the cells or even animal pathogens. Fetal bovine serum (FBS) is widely used, at least in some stages of the culture of RPE cells. KnockOut™ Serum Replacement (KO-SR), used to replace FBS in many laboratories, still contains BSA (BSA) and bovine transferrin. In addition, most of the current differentiation methods utilize a culture environment produced by mouse embryonic fibroblast (MEF) cells widely used as feeder cells for hESCs and hiPSCs.

WO 2010/144696 relates to methods and compositions for producing neural cells from stem cells, while WO 2012/103012 relates to generating inner ear cells. Among potential differentiation-inducing agents, both publications disclosed bFGF, a TGF-beta inhibitor, and a Wnt inhibitor.

There still is need for supplies and methods for culturing stem cells via controlled differentiation to produce cells contributing to treatment and research of retinal and corneal epithelium conditions, diseases, pathologies, as well as toxicology and drug development. Further, cell culture media for use in such methods are equally desired. Further, avoidance of undefined components, such as amniotic membrane or conditioned medium is also an important aim.

### BRIEF DESCRIPTION OF THE INVENTION

It is thus an object of the present invention to provide means and methods effective to differentiate stem cells into eye precursor cells and further into corneal epithelial precursor cells, which may then be maturated into mature corneal epithelial cells or into corneal stratified epithelium.

Thus, in one aspect, the present invention provides a method of producing differentiated eye cells selected from the group consisting of corneal epithelial precursor cells, corneal epithelial cells and stratified corneal epithelium. Said method comprises a) culturing pluripotent stem cells in an induction medium comprising TGF-beta inhibitor, a Wnt inhibitor and a fibroblast growth factor, thereby producing eye precursor cells; b) culturing the eye precursor cells obtained in step a) in a cell culture medium comprising one or more supplements selected from the group consisting of epidermal growth factor (EGF), hydrocortisone, insulin, isoproterenol, and tri-iodo-thyronine, wherein the medium does not contain any of the following supplements: a TGF-beta inhibitor, a Wnt inhibitor and a fibroblast growth factor, thereby producing corneal epithelial precursor cells, wherein said corneal epithelial precursor cells express corneal epithelial marker p63, said expression being preferably quantified with immunofluorescent staining; an optionally maturating said corneal epithelial precursor cells into mature corneal epithelial cells or into corneal stratified epithelium.

Other aspects, specific embodiments, objects, details, and ad-vantages of the invention are set forth in the dependent claims and will be-come apparent from the following drawings, detailed description and examples.

### BRIEF DESCRIPTION OF THE DRAWINGS

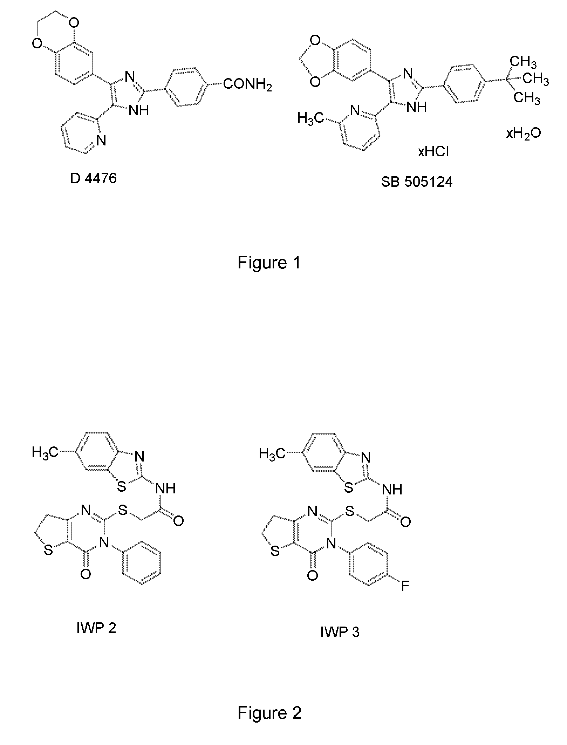

In the following, invention will be described in greater detail by means of preferred embodiments with reference to the attached drawings, in which
Figure 1 exemplifies structures of two commercial small molecular TGF-b1-inhibitors (D 4476 and SB 505124).
Figure 2 exemplifies two commercially available small molecular Wnt-inhibitor structures (IWP-2 and IWP-3).

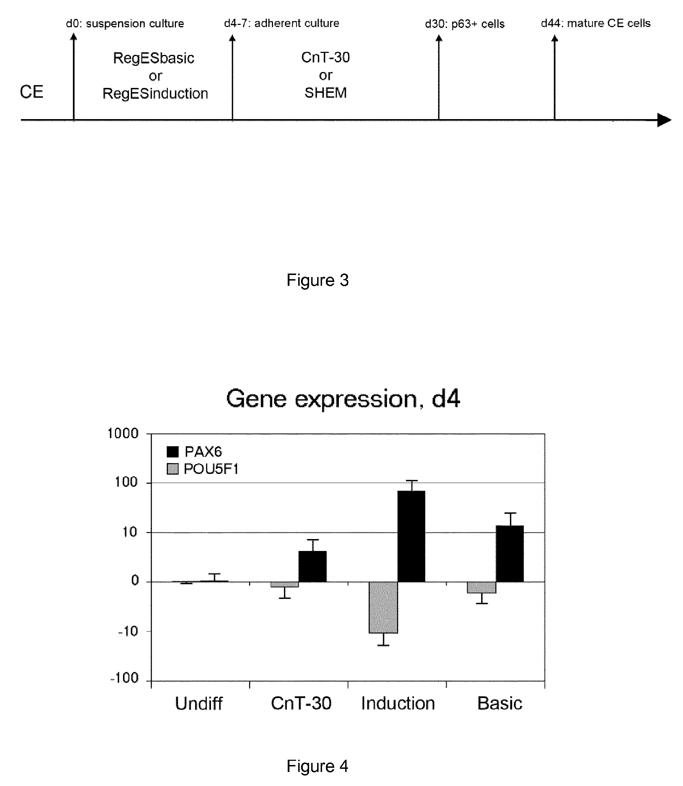

Figure 3 gives an overview on the strategy of the experiments conducted to validate the present method. It explains as function of time the two stages of cultivation, i.e. the induction stage and differentiation stage, for producing corneal epithelial (CE) linages, and points out the key time-points. It also clearly shows the media studied in each test.
Figure 4 illustrates differentiation results obtained after 4 days in the induction stage, carried out in different culture media. From left to right: sample of undifferentiated stem cells as control (Undiff); commercial medium marketed for cultivation of corneal epithelial cells (CnT-30); RegES medium modified according to the present invention to comprise a TGF-beta inhibitor, a Wnt-inhibitor and a fibroblast growth factor (RegESinduction); unsupplemented RegES medium (RegESbasic). Differentiation was followed measuring two markers, *POU5F1,* which is present in undifferentiated cells and *PAX6,* which indicates differentiation into eye specific cell lineages.

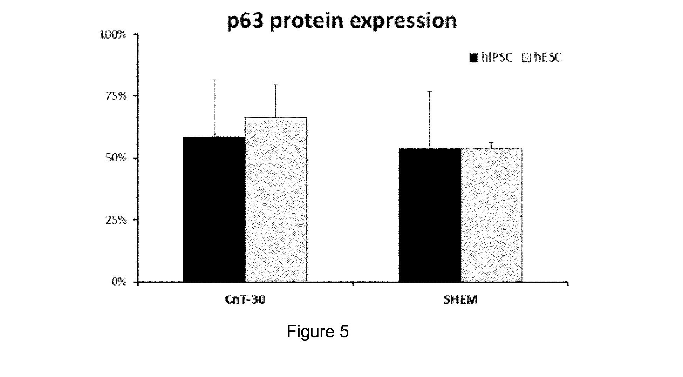

Figure 5 illustrates the outcome of an embodiment of the present differentiation method, wherein human induced pluripotent stem cells (hiPSC) or human embryonic stem cells (hESC) were differentiated into corneal epithelial precursor cells. The maturation stage was carried out either in commercially available CnT-30 medium or SHEM medium containing EGF, hydrocortisone, insulin, isoproterenol and tri-iodo-thyronine. Corresponding results were obtained regardless of the maturation medium and cell type used.

### DETAILED DESCRIPTION OF THE INVENTION

In some aspects, the present invention provides a two-stage differentiation method comprising an induction stage and a further differentiation and maturation stage for differentiating said eye precursor cells into differentiated eye cells, such as corneal epithelial precursor cells, and further into mature corneal epithelial cells. As used herein, the terms "precursor" and "progenitor" may be used interchangeably unless otherwise indicated.

### Induction stage and medium

The present induction stage is aimed at inducing pluripotent stem cells towards surface ectoderm and eye precursor cells by subjecting said stem cells, preferably in a suspension culture, to an induction medium comprising active "induction supplements", i.e. a TGF-beta inhibitor, a Wnt inhibitor and a fibroblast growth factor. Both said induction stage and said induction medium are disclosed herein.

If the induction stage is to be carried out in adherent culture, it is advantageous to use substrates coated with extracellular matrix (ECM) proteins as generally known in the art and discussed in more detail below under "differentiation method and media".

As used herein, the term "pluripotent stem cell" refers to any stem cell having the potential to differentiate into all cell types of a human or animal body, not including extra-embryonic tissues. These stem cells include both embryonic stem cells (ESCs) and induced pluripotent cells (iPSCs). Hence, the cells suitable for the method of the present invention include stem cells selected from iPSCs and ESCs.

ESCs are of great therapeutic interest because they are capable of indefinite proliferation in culture and are thus capable of supplying cells and tissues for replacement of failing or defective human tissue. Producing eye precursor cells from human embryonic stem cells may meet ethical challenges. Human embryonic stem cells may be used with the proviso that the method itself or any related acts do not include destruction of human embryos.

Induced pluripotent stem cells, commonly abbreviated as iPS cells or iPSCs are a type of pluripotent stem cell artificially derived from a non-pluripotent cell - typically an adult somatic cell - by inducing a forced expression of specific genes. One benefit of use of iPS cells is avoidance of the involvement embryonic cells altogether, and hence any ethical questions thereof. Therefore, according to another embodiment of the present invention, when producing eye precursor cells, use of iPS cells is preferred.

Induced pluripotent stem cells are similar to natural pluripotent stem cells, such as embryonic stem cells, in many aspects, such as the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed. Induced pluripotent cells are typically made from adult skin cells, blood cells, stomach, or liver, although other alternatives may be possible. A man skilled in the art is familiar with research and therapy potential of iPS cells, i.e. from publication of Bilic and Izpiua Belmonte (2012).

As used herein the term "xeno-free" refers to absence of any foreign material or components. Thus, in case of human cell culture, this refers to conditions free from non-human animal components. In other words, when xeno-free conditions are desired for production of eye precursor cells, or any cells maturated therefrom, for human use, ES or iPS cells are selected to be of human origin.

As used herein, the term "eye precursor cell" refers broadly to any cell lineage of the eye induced from pluripotent stem cells using the present induction method and/or induction medium. Eye precursor cells are characterized by down-regulation of the pluripotency marker *OCT-4* (also known as *POU5F1)* and up-regulation of *PAX6,* a gene indicating differentiation into eye specific cell lineages.

In the present induction stage, pluripotent stem cells are cultured in the present induction medium for a period of time which may vary depending on different variables such as the final composition of the induction medium and the purpose of the induction method. Typically, the duration of the induction may vary from a couple of days to several days. A preferred time range is from about four days to about seven days, i.e. from about 96 hours to about 168 hours. As used herein, the term "about" refers to a variation of 10 percent of the value specified. Thus, the term "about four days" carries a variation from 87 to 105 hours, while the term "about seven days" carries a variation from 152 to 186 hours. If an induction time shorter than 4 to 7 days is used, down-regulation of *OCT4* and up-regulation of *PAX6* is weak leading to less efficient differentiation as determined by weak expression of precursor markers PAX6 and p63 Moreover, if an induction time longer than 4 to 7 days is used, more neural differentiation can be expected, because human pluripotent stem cells have a known tendency to differentiate towards neural lineages, especially in the presence of basic fibroblast growth factor.

As set forth above, the present induction medium comprises a TGF-beta inhibitor, a Wnt inhibitor and a fibroblast growth factor as active supplements. These induction supplements were found to enhance differentiation of pluripotent stem cells towards eye precursor cells and improve their further differentiation efficiency into clinically valuable eye cells, such as corneal epithelial precursor cells, corneal epithelial cells, and stratified corneal epithelia.

The present induction medium may be considered to consist of or comprise a basal medium and the present induction supplements. However, further supplements common in the art may be applied. As used herein, the term "common cell culture supplements" refers to ingredients used in practically every cell culture medium including antibiotics, L-glutamine, and serum, serum albumin or a serum replacement, preferably a defined serum replacement.

On the other hand, in some embodiments, the induction medium does not contain ingredients other than the induction supplements, basal medium, antibiotics, L-glutamine, and a defined serum replacement. In some further embodiments, the induction supplements are a TGF-beta inhibitor of Formula I or II, a Wnt inhibitor according to Formula III, and bFGF. In a still further embodiment, the induction supplements are SB-505124, IWP-2, and bFGF.

Any of the aforementioned embodiments may form a basis for additional or alternative embodiments, wherein the induction medium does not comprise any supplements generally known to be inductive for differentiation types other than differentiation towards eye lineages, such types as neural differentiation. Such generally known supplements include, but are not limited to, retinoic acid, ascorbic acid, brain-derived neurotrophic factor BDNF, and glial-derived neurotropichic factor GDNF.

The basal medium may be any stem cell culture medium in which stem cells can effectively be differentiated. In a preferred embodiment, the basal medium is RegESbasic medium. In other preferred embodiments, the basal medium is RPEbasic. According to further preferred embodiment, the induction medium is xeno-free, serum-free or defined, more preferably a combination of these and most preferably xeno-free, serum-free and defined at the same time. These terms are defined below.

In a preferred composition of the induction medium, the content of the TGF-beta inhibitor is from 1 µM to 100 µM, preferably from 1 to 30 µM, the Wnt-inhibitor is from 1 µM to 100 µM, preferably from 1 µM to 30 µM, and the content of fibroblast growth factor is from 1 ng/ml to about 1000 ng/ml, preferably about 2 ng/ml to about 100 ng/ml, and more preferably about 30 ng/ml to about 80 ng/ml.

### TGF-beta (TGF-β) inhibitor

As used herein, with "TGF-beta inhibitor" is referred functionally to a substance capable of inhibiting transforming growth factor β1.

Transforming growth factor β1 (TGF-β1) is a member of a large superfamily of pleiotropic cytokines that are involved in many biological activities, including growth, differentiation, migration, cell survival, and adhesion in diseased and normal states. Nearly 30 members have been identified in this superfamily. These are considered to fall into two major branches: TGFb/Activin/Nodal and BMP/GDF (Bone Morphogenetic Protein/Growth and Differentiation Factor). They have very diverse and often complementary functions. Some are expressed only for short periods during embryonic development and/or only in restricted cell types (e.g. anti-Mullerian hormone, AMH, Inhibin) while others are widespread during embryogenesis and in adult tissues (e.g. TGFβ1 and BMP4). TGF-β1 is a potent regulator in the synthesis of the extracellular matrix (fibrotic factor) and plays a role in wound healing.

In chemical and structural terms, suitable TGF-beta inhibitory function may be found among proteins and small organic molecules. A man skilled in the art is aware of means for isolating proteins from biological matrixes or producing them i.e. by recombinant techniques.

Compounds exhibiting TGF-beta inhibitory activity may be found by screening. Preferably a TGF-beta inhibitor is an organic molecule having a relatively low molar mass, e.g a small molecule having molar mass less than 800 g/mol, preferably less than 500 g/mol. As a general structure, Formula I, a suitable low molar mass TGF-inhibitor may be described as: 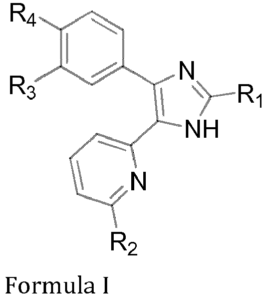 wherein R₁ represents an C₁-C₅ aliphatic alkyl group, carboxylic acid, amide, and R₂ represents an C₁-C₅ aliphatic alkyl, R₃ and R₄ represent aliphatic alkyls including heteroatoms, O or N, which may be linked together to form a 5- or 6 member heteroring.

A typical structure comprises a hetero ring having 2 oxygen atoms, when it can be referred to as a small molecule of general formula II: 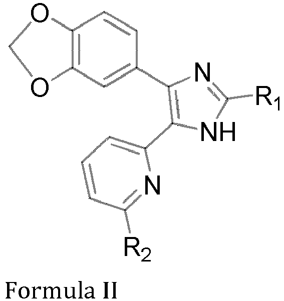 wherein, R₁ represents an C₁-C₅ aliphatic alkyl group, an aromatic carboxylic acid or amide, and R₂ represents an C₁-C₅ aliphatic alkyl.

One example of such an TGF-b1-inhibitor is 4-[4-(1,3-Benzodioxol-5-yl)-5-(2-pyridinyl)-1H-imidazol-2-yl]-benzamide; 4-[4-(3,4-Methylenedioxyphenyl)-5-(2-pyridyl)-1H-imidazol-2-yl]-benzamide; 4-(5-benzol[1,3]dioxol-5-yl-4-pyridin-2-yl-1H-imidazol-2-yl)-benzamide hydrate, with the chemical formula in figure 1, which is commercially available from suppliers and marketed as a selective inhibitor of transforming growth factors type I receptor (ALK5), ALK4 and. Selectively inhibits signaling from TGF-βand activin; does not inhibit other ALK family members. Another example of TGF-inhibitors is 2-(5-benzo[1,3]dioxol-5-yl-2-tert-butyl-3H-imidazol-4-yl)-6-methylpyridine hydrochloride hydrate, the structure of which is given in figure 1 as well.

However, other small molecules exhibiting TGF-beta inhibitory activity or commercially marketed as TGF-inhibitors may be equally suitable in the context of the present invention. When selecting said TGF-beta inhibitor from substances obtainable by chemical synthesis or recombinant production, a defined medium can be provided. It also complies with requirements of xeno-free and serum-free conditions.

### Wnt- inhibitor

As used herein, a "Wnt inhibitor" refers to a substance capable of inhibiting Wnt signaling pathway. More specifically Wnt-inhibiting functions relate to i.e. preventing palmitylation of Wnt proteins by porcupine (Porc), a membrane-bound O-acyltransferase, thereby blocking Wnt secretion and activity. A Wnt inhibitor also blocks phosphorylation of the Lrp6 receptor and accumulation of both Dvl2 and β-catenin 1. The inhibition of the Wnt pathway through the use of a Wnt inhibitor has also been shown to promote the formation of cardiomyocytes from human embryonic stem cell-derived mesodermal cells.

Both protein and small molecular Wnt inhibitors are known in the art. In context of the present invention, small molecular Wnt inhibitors are preferred.

When selecting said Wnt inhibitor from substances obtainable by chemical synthesis, it contributes to providing a defined medium. Suitable compounds have been discovered by screening a group of organic molecules. Preferably such an inhibitor is an organic molecule having a relatively low molar mass, e.g. a small molecule having molar mass less than 800 g/mol, preferably less than 500 g/mol. As a general structure, Formula III, a suitable small molecular Wnt inhibitor may be described as: 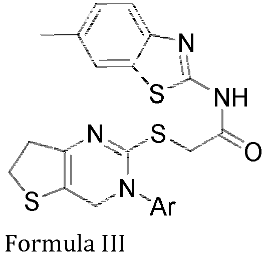 wherein Ar refers to substituted or unsubstituted aryl group.

Commercially known Wnt inhibitors are exemplified in figure 2.

### Fibroblastgrowth factor

In the medium of the present invention, a fibroblast growth factor is required to contribute to the differentiation. Fibroblast growth factors, or FGFs, are a family of growth factors generally involved in angiogenesis, wound healing, and embryonic development. The FGFs are heparin-binding proteins and interactions with cell-surface-associated heparan sulfate proteoglycans have been shown to be essential for FGF signal transduction. FGFs are key players in the processes of proliferation and differentiation of wide variety of cells and tissues.

Fibroblast growth factors suitable for use in the present invention include fibroblast growth factors (FGFs) such as basic FGF (bFGF or FGF-2). While FGF is preferably used, other materials, such as certain synthetic small peptides (e.g. produced by recombinant DNA variants or mutants) designed to activate fibroblast growth factor receptors, may be used instead of FGF. Fibroblast growth factors may be included in the serum replacement used as basal medium or they may be added separately to the final cell culture medium according to the present invention.

### Differentiation method and media

The present differentiation method is a two-stage method comprising the above-described induction stage, preferably but not necessarily carried out in a suspension culture, followed by a differentiation stage in adherent culture. In the latter stage, eye precursor cells produced in the induction stage are differentiated into corneal epithelial precursor cells.

In some other embodiments, the present differentiation method is a three-stage method comprising the above-described induction stage, preferably but not necessarily carried out in suspension culture, followed by a differentiation stage and a further maturation stage in adherent culture. In the maturation stage, corneal epithelial precursor cells are maturated into mature corneal epithelial cells or even into corneal stratified epithelia.

In practice, the differentiation stage and maturation stage are carried out successively in the same way; they only differ from each other in respect of timing, as explained in more detail below. Even the culture medium to be used in these stages is the same. Thus, the terms "differentiation medium" and "maturation medium" are interchangeable. The same applies to the terms "differentiation supplements" and "maturation supplements". If desired, a shift from a differentiation stage to a maturation stage may concern only a selected subpopulation of cells obtained from the differentiation stage.

Preferably, the differentiation and maturation conditions are xeno-free, serum-free or defined, more preferably a combination of these, and most preferably xeno-free, serum-free and defined at the same time.

Since the differentiation and maturation stages are to be performed in an adherent culture and the ability to attach to extracellular matrix (ECM) is considered to be important for epithelial cells, it is advantageous to use substrates, such as cell culture plates or bottles, coated with ECM proteins as generally known in the art. In fact, adhesion to collagen IV is generally used for selecting epithelial precursor cells from limbal epithelial cell populations and differentiating pluripotent stem cells. Thus, in some preferred embodiments, the cell culture substrate is coated with collagen IV. Other non-limiting examples of suitable coating materials include collagen I, laminin, vitronectin, fibronectin, and Matrigel™. When xeno-free conditions are desired for human use, the substrate is to be coated with an ECM protein of human origin. Means and methods for coating cell culture substrates are generally available in the art.

### Corneal epithelial differentiation

In the present invention, the eye precursor cells obtainable by the present induction stage are differentiated further into corneal epithelial precursor cells. This method may be termed as a two-stage differentiation method.

In this context, the term "corneal epithelial precursor cells" refers to cells which are positive for, i.e. express, corneal epithelial marker p63, which may be quantified with the help of immunofluorecent staining. According to an embodiment, the corneal epithelial precursor cells expressing said marker p63 represent at least 65%, preferably at least 75%, most preferably at least 90% of the total cell population. Population of corneal epithelial precursor cells may be used clinically.

Typically, obtaining corneal epithelial precursor cells requires culturing eye precursor cells under the present corneal differentiation conditions for about 10 to about 35 days, preferably for about 25 days. In some preferred embodiments, corneal epithelial precursor cells are obtained by carrying out the induction stage for about four to about seven days followed by the corneal differentiation stage for about 23 to 26 days. The most pure p63-positive cell population can be obtained after a differentiation stage of this length. Shorter differentiation time yields more heterogeneous cell populations, while longer differentiation time results in terminal maturation towards corneal epithelial cells.

In some further embodiments, said corneal epithelial precursor cells may be maturated even further into mature corneal epithelial cells or stratified corneal epithelia, as demonstrated by a characteristic marker expression and morphology. Such further maturation may be obtained by continued cell culturing, typically for an additional 10 to 20 days, in the present corneal maturation conditions, which in practice correspond to the corneal differentiation conditions. This embodiment may be termed as a three-stage differentiation method.

A suitable culture medium for use in the differentiation and maturation stages may be, for instance, any available corneal medium such as CnT-30 which is commercially available from CELLnTECH, or any supplemental hormonal epithelial medium (SHEM) suitable for culturing corneal epithelial cells. In some other embodiments, the differentiation and maturation medium may be composed by adding ingredients such as one or more differentiation and maturation supplements selected from the group consisting of epidermal growth factor (EGF), hydrocortisone, insulin, isoproterenol and tri-iodo-thyronine, into any suitable basal medium. In some embodiments, the corneal differentiation and maturation medium does not contain ingredients other than said one or more differentiation and maturation supplements, basal medium, antibiotics, L-glutamine, and a defined serum replacement. In some further embodiments, the basal medium is a 1:1 mixture of DMEM and Ham's F12 nutrient mixture.

Any of the aforementioned embodiments may form a basis for additional or alternative embodiments, wherein the differentiation and maturation medium does not comprise any supplements, which are generally known to cause differentiation towards cells types other than eye cells, such types as neural differentiation. Such generally known supplements include, but are not limited to, retinoic acid, ascorbic acid, brain-derived neurotrophic factor BDNF, and glialderived neurotropichic factor GDNF.

Importantly, differentiation of pluripotent stem cells into corneal epithelial precursor cells and further maturation into corneal epithelial cells, or stratified corneal epithelia was significantly better when the cells were differentiated by the present induction method followed by the present corneal differentiation and maturation stage than when performing the corneal differentiation and maturation stage without prior induction. Improved results were obtained e.g. regarding better adhesion to collagen IV-coated substrates, uniform corneal epithelial cell morphology, and enhanced expression of p63, as well as other key markers (most notably cytokeratins 3, 12 and 15) both on the gene and protein level.

### General features of present media

The culture medium may be considered to consist of basal medium and supplements. In the present induction medium, the three essential supplements are TGF-beta inhibitor, a Wnt inhibitor and a fibroblast growth factor. In the present corneal epithelial differentiation and maturation medium, exemplary or preferred supplements additional to common cell culture supplements are selected from the group consisting of EGF, hydrocortisone, insulin, isoproterenol, and tri-iodo-thyronine. In the present RPE differentiation and maturation medium, exemplary or preferred supplements additional to common cell culture supplements are selected from the group consisting of taurine, hydrocortisone, tri-iodo-thyronine, activin A, insulin, transferrin, sodium selenite, putrescine, and progesterone. However, in the context culture media, further supplements common in the art may be applied, unless they are known to direct differentiation towards tissues other than the eye. When referring to components of a medium, the term includes both supplements and ingredients to the basal medium.

When in use or when ready for use, the present culture media comprise appropriate essential supplements set forth above. However, according to common practice in the field, the ingredients for a medium may be provided as a concentrate comprising said components or a set of vials from which suitable combination is prepared in a laboratory according to instructions provided.

As referred here, "culture medium" or "cultivation medium" refers broadly to any liquid or gel formulation designed to support the growth of microorganisms, cells or small plants. When referring to formulation designed for cell maintenance and growth, term "cell culture medium" is used. In the art, expressions such as induction medium, growth medium, differentiation medium, maturation medium etc. can be considered as subspecies to general expression culture medium. A man skilled in the art is familiar with the basic components necessary to maintain and nourish the living subjects in or on the culture medium, and commercial basic media are widely available. Typically such basic components are referred to as "basal medium", which contains necessary amino acids, minerals, vitamins and organic compounds. Generally, it can be obtained from biological sources, such as serum or be combined from isolated and pure ingredients. If desired, basal medium may be supplemented with substances contributing to special features or functions of culture medium. Very common supplements include antibiotics, which are used to limit growth of contaminants.

Non-limiting examples of suitable basal media to be used in the present cell culture media of various embodiments include KnockOut Dulbecco's Modified Eagle's Medium (KO-DMEM), Dulbecco's Modified Eagle's Medium (DMEM), Minimal Essential Medium (MEM), Basal Medium Eagle (BME), RPMI 1640, F-10, F-12, Glasgow's Minimal Essential Medium (G-MEM), Iscove's Modified Dulbecco's Medium and any combinations thereof. In some preferred embodiments, RegES medium altered by omitting retinol, bFGF and activin A (referred to herein as RegESbasic) is used as a basal medium. In some more preferred embodiments, RegESbasic is used as a basal medium in the present induction medium.

For better clinical acceptance all culture media used herein are preferably substantially xeno-free, substantially serum-free or defined, more preferably combinations of these and most preferably xeno-free, serum-free and defined at the same time. With substantially is meant here that unintentional traces are irrelevant and what is under clinical or laboratory regulations considered and accepted as xeno-free, serum-free or defined, applies here as well.

Traditionally, serum, especially fetal bovine serum (FBS) has been valued in cell cultures providing essential growth and survival components for *in vitro* cell culture of eukaryotic cells. It is produced from blood collected at commercial slaughterhouses from cattle bred to supply meat destined for human consumption. "Serum free" indicates that the culture medium contains no serum, either animal or human. Defined medium is valuated when there are contradictions for use of undefined media, e.g. "conditioned medium", which refers to spent media harvested from cultured cells containing metabolites, growth factors, and extracellular matrix proteins secreted into the medium by the cultured cells. Undefined media may be subject to considerable dissimilarities due to natural variation in biology. Undefined components in a cell culture compromise the repeatability of cell model experiments e.g. in drug discovery and toxicology studies. Hence, "defined medium" or "defined culture medium" refers to a composition, wherein the medium has known quantities of all ingredients. Typically, serum that would normally be added to culture medium for cell culture is replaced by known quantities of serum components, such as, e.g., albumin, insulin, transferrin and possibly specific growth factors (i.e., basic fibroblast growth factor, transforming growth factor or platelet-derived growth factor).

A chemically defined medium is a growth medium suitable for the in vitro cell culture of human or animal cells in which all of the chemical components are known. A chemically defined medium is entirely free of animal-derived components and represents the purest and most consistent cell culture environment. By definition chemically defined media cannot contain fetal bovine serum, bovine serum albumin or human serum albumin as these products are derived from bovine or human sources and contain complex mixes of albumins and lipids.

Chemically defined media differ from serum-free media in that bovine serum albumin or human serum albumin with either a chemically defined recombinant version (which lacks the albumin associated lipids) or synthetic chemical such as the polymer polyvinyl alcohol which can reproduce some of the functions of BSA/HSA. The next level of defined media, below chemically defined media is protein-free media. These media contain animal protein hydrolysates and are complex to formulate although are commonly used for insect or CHO cell culture.

According to some embodiments, the present media comprises a xeno-free serum replacement formulation. A defined xeno-free serum replacement formulation or composition may be used to supplement any suitable basal medium for use in the in vitro derivation, maintenance, proliferation, or differentiation of stem cells. Said serum replacement may be used to supplement either serum-free or serum-containing basal mediums, or any combinations thereof. When xeno-free basal medium is supplemented with the present xeno-free serum replacement, the final culture medium is xeno-free as well. One example is described in Rajala et al. 2010 which is incorporated here as reference describing a xeno-free serum replacement applicable in the context of the present invention. Another non-limiting example of a defined serum replacement is KnockOut™ Serum Replacement (Ko-SR) commercially available from Life Technologies.

A man skilled in the art is familiar with different culture medium concentrations. Often, culture medium is diluted and prepared to the final composition immediately before use. Therefore, it is understood that any stock solution or preparation kit suitable for use in such immediate preparation is included in the scope of the present invention as well. For example, for preparation of a culture medium according to present description, a cell culture kit comprises the TGF-beta inhibitor, the Wnt-inhibitor and the fibroblast growth factor each in a separate container or any combinations thereof as supplements, and optionally other components, such as basal medium or supplies for preparation thereof, as well.

### Reference experimental part

### MATERIALS AND METHODS

### Cell line and cell culture media

Undifferentiated pluripotent stem cell line was derived and characterized as described previously (Skottman 2010). The cell line was cultured on mitotically inactivated human foreskin fibroblast (hFF) feeder cells (CRL-2429, ATCC). Undifferentiated pluripotent stem cells were maintained in a culture medium consisting of Knock-Out Dulbecco's Modified Eagle Medium (KO-DMEM) supplemented with 20% Knock-Out Serum Replacement (KO-SR), 2 mM Gluta-Max-I, 0.1 mM 2-mercaptoethanol (all from Invitrogen), 1% non-essential amino acids (NEAA), 50 U/ml penicillin/streptomycin (both from Lonza Group Ltd) and 8 ng/ml human basic fibroblast growth factor (bFGF, PeproTech). The culture medium was changed five times a week and undifferentiated colonies were enzymatically passaged onto fresh feeder cell layers at ten-day intervals.

Three different media were used for corneal epithelial differentiation: A) Commercially-available defined and serum-free CnT-30 corneal epithelium medium (CELLnTEC Advanced Cell Systems) supplemented with the appropriate supplements provided with the medium and 50 U/ml penicillin/streptomycin. B) Serum-free and xeno-free RegES medium originally developed for the culture of undifferentiated stem cells (Rajala et al. 2010). The medium composition was altered by omitting retinol, bFGF and activin A, and the resulting differentiation medium is referred to as RegESbasic medium. The composition of said RegESbasic medium was as given in Table 1. C) RegESbasic medium supplemented with 10 µM TGF-beta inhibitor (SB-505124 Sigma), 10 µM Wnt-inhibitor (IWP-2 Merck Biosciences) and 50 ng/ml bFGF and the resulting induction medium is referred to as RegESinduction medium.

**Table 1. RegESbasic medium representing an example of xeno-free cell culture medium according to Rajala et al., 2010.**

| **Component** | **Concentration (mg/L)** | **Manufacturer** |
|---|---|---|
| **Amino acids** | | |
| Glycine | 53 | Sigma |
| L-histidine | 183 | Sigma |
| L-isoleucine | 615 | Sigma |
| L-methionine | 44 | Sigma |
| L-phenylalanine | 336 | Sigma |
| L-proline | 600 | Sigma |
| L-hydroxyproline | 15 | Sigma |
| L-serine | 162 | Sigma |
| L-threonine | 425 | Sigma |
| L-tryptophan | 82 | Sigma |
| L-tyrosine | 84 | Sigma |
| L-valine | 454 | Sigma |

| **Vitamins, antioxidants and trace elements** | | |
|---|---|---|
| Ascorbic acid | 50 | Sigma |
| Glutathione | 1.5 | Sigma |
| Selenium | 1x10⁻⁵ | Sigma |
| Thiamine | 9 | Sigma |
| Trace elements B | 1:1000 | Cellgro |
| Trace elements C | 1:1000 | Cellgro |

| **Proteins** | | |
|---|---|---|
| Human serum albumin* | 10000 | Sigma |
| Insulin | 100 | Invitrogen |
| Transferrin | 8 | Sigma |

| **Other components** | | |
|---|---|---|
| NEAA (100x | 1% | Lonza |
| Glutamax-I | 2 mM | Invitrogen |
| β-mercaptoethanol | 0.1 mM | Invitrogen |

| | | |
|---|---|---|
| Basal medium: KO-DMEM (Invitrogen) *To provide better consistency and less variation, HSA can be substituted with recombinant HSA. | | |

### Eye precursor induction

The experimental design is schematically presented in Figure 3. To initiate eye precursor differentiation of pluripotent stem cells, undifferentiated colonies were manually dissected and transferred to suspension culture in 6-well plates (Corning ultra-low attachment). Cells were cultured as three-dimensional cell clusters for four to seven days, changing the culture medium daily. This induction stage was carried out in RegESinduction (cornea epithelial differentiation) and in compared control conditions without inductive molecules. It would have also been possible to perform the induction by using RPEinduction for corneal epithelial differentiation.

### Corneal epithelial differentiation and maturation

After the induction stage, cell clusters were plated onto cell culture substrate coated with human placental collagen IV (5 µg/cm², Sigma) at a density of about 50 clusters/cm². Either 24-well plates (Corning CellBIND) or 24-well hanging cell culture inserts (Millipore, 1 µm pore size) were used. Cells were maintained in adherent culture for 40 more days in either CnT-30 medium (conditions A-C) or RegESbasic medium (control), replacing the culture medium three times a week. Alternatively, CnT-30 medium was replaced with supplemental hormonal epithelial medium (SHEM), consisting of DMEM/F12 basal medium supplemented with 15% KO-SR, 2 mM GlutaMax-I, 100 µg/ml hydrocortisone (Sigma), 10 µg/ml insulin (Invitrogen), 0.25 µg/ml isoproterenol (Sigma), 1.35 ng/ml tri-iodo-thyronine (Sigma), 10 ng/ml EGF (PeproTech) and 50 U/ml penicillin/streptomycin.

### Quantitative real time polymerase chain reaction (qPCR)

Total RNA was extracted from undifferentiated pluripotent stem cells, and from cell aggregates collected after the induction phase, using NucleoSpin RNA II kit (Macherey-Nagel, GmbH & Co). The RNA concentration of each sample was determined using the NanoDrop-1000 spectrophotometer (NanoDrop Technologies). From each RNA sample, 200 ng were used to synthesize cDNA using the high-capacity cDNA RT kit (Applied Biosystems). The resulting cDNA samples were analyzed with qPCR using sequence-specific TaqMan Gene Expression Assays (Applied Biosystems) for *POU5F1* (Hs00999632_g1) and *PAX6* (Hs01088112_m1). All samples and controls were run as triplicate reactions with the 7300 Real-time PCR system. Results were analyzed with the 7300 System SDS Software (Applied Biosystems) and Microsoft Excel. Based on the cycle threshold (CT) values given by the software, the relative quantification of each gene was calculated by applying the -2ΔΔCt method. Results were normalized to *GAPDH* (Hs99999905_m1), with the undifferentiated pluripotent stem cells as the calibrator to determine the relative quantities (RQ) of gene expression in each sample. The analysis was repeated four times.

### Immunofluorescence

To assess corneal epithelial differentiation, protein expression of p63 was analyzed and quantified at three time-points (10, 20 and 30 days in differentiation culture) from cells cultured on collagen IV-coated hanging inserts. Cells were fixed with 4% paraformaldehyde (Sigma) for 20 minutes and cell membranes permeabilized with 0.1% Triton-X-100 for 10 minutes. Non-specific binding sites were blocked with 3% BSA for 1 hour. The goat anti-p63 primary antibody (Santa Cruz) was diluted 1:100 in 0.5% BSA, and incubated with the cells for 1 hour at room temperature, or overnight at +4. Primary antibody detection was done with Alexa Fluor 568-conjugated donkey anti-goat secondary antibody (Molecular Probes) diluted 1:800 in 0.5% BSA for 1 hour at room temperature. Samples were mounted onto object glasses in VectaShield mounting medium containing DAPI for visualization of nuclei. Images were captured with fluorescence microscope from multiple randomly selected areas. Total cell numbers were determined by counting the number of DAPI-stained nuclei, and percentages of p63-positive nuclei were consequently quantified. At the end-point of the study, a total of 44 days in differentiation culture, expression of several proteins was analyzed with immunofluorescence. The membranes of hanging cell culture inserts were cut into several pieces and treated in the way described above. Binding of the primary antibodies used was visualized using secondary antibodies against the appropriate species labeled with Alexa Fluor 488 or 568.

Cells cultured on 24-well plates were used for quantitative immunofluorescence analysis at the end-point of the study. Cells were rinsed with PBS and detached using TrypLE Select (Gibco) for 5 minutes at +37. Cell suspensions were strained through 40 µm cell strainers and centrifuged at 1500 rpm for 5 minutes. Cell pellets were resuspended in cold PBS, and single-cell suspension volumes adjusted to contain 50 000 cells/150 µl sample. Cells were then spun onto object glasses using a cytocentrifuge (CellSpin II). Immunofluorescence stainings were performed for the resulting cytospin samples as described above. Images of multiple randomly selected areas were captured with a fluorescence microscope. Percentages of cells positive for each marker were quantified in relation to DAPI-stained cells. This analysis was repeated three times.

### RESULTS

### Inhibition of the TGF-β and Wnt signaling pathways directs pluripotent stem cell differentiation towards eye specific lineages

To study the effects of induction medium on early-stage differentiation, expression of *POU5F1*, a marker of undifferentiated cells, and *PAX6,* a gene important for eye development, was studied using qPCR. Results are shown in figure 4. After the four-day induction phase in suspension culture, expression of *POU5F1* decreased in all conditions, while expression of *PAX6* increased. The differences in expression levels compared to undifferentiated cells were significantly greater (p<0.05, determined with Mann-Whitney U tests) in cells that underwent induction with SB-505124, IWP-2 and bFGF, than in the other induction media, suggesting higher extent of differentiation in these conditions.

### Small molecule induction increases corneal epithelial differentiation efficiency and reproducibility

Protein expression of the commonly-used corneal epithelial progenitor marker p63 was analyzed at ten-day intervals by means of immunofluorescence staining. The first time-point was after six days in adherent culture, a total of ten days in differentiation culture, giving the cell clusters time to properly adhere to the collagen IV-coated substrate. The next time-point was at day 20, and the last one at day 30. Expression of p63 was not detected in cells cultured under spontaneous differentiation conditions in RegESbasic throughout the course of the study, suggesting that corneal epithelial precursor cell differentiation does not take place spontaneously. Cells maintained in CnT-30 medium expressed p63 at each time-point, and expression levels were clearly affected by the induction medium. In order to quantify the differences between the culture conditions, amounts of p63-positive cells were counted at each time-point. After induction with CnT-30, p63 expression was quite varied between biological replicates, masking the overall differences between time-points. In contrast, after induction with RegESinduction medium, the number of p63-positive cells increased with time from about 50% at day 10, to about 90% at day 30, with the least interreplicate variation of all the studied conditions. The corneal precursor cell population after 30 culture demonstrated thus interestingly high degree of differentiation. Induction with RegESbasic medium resulted in the lowest p63 expression levels - on average 25-50% throughout the course of the study, with fairly high variation between replicates.

Maturation in SHEM yielded comparable amounts of p63-positive cells after a total of 20 days in differentiation culture, as verified by immunofluorescence staining (Figure 5).

### Corneal epithelial cells can be differentiated from pluripotent stem cells

Because induction medium affected the subsequent yield of p63-positive corneal epithelial precursor cells, expression of several proteins typical to the corneal precursors (p63 and CK15) and mature epithelium (CK3 and CK12) was quantified also at the end-point of the study.

After a total of 44 days in differentiation culture, cells were analyzed with immunofluorescence, staining positive for progenitor markers p63 and CK15, and markers specific to corneal epithelium, CK3 and CK12. The results showing percentages of differentiation are compiled in Table 2, where ratio between differentiated cells and total number of cells is given, the scales indicating that 0% means that none of the cells (of the population studied) express the differentiation marker, and 100% means that all of the cells express the differentiation marker. The two markers of mature corneal epithelium were detected especially in stratified regions, suggesting that stratification is required for full maturation of corneal epithelium. Expression of progenitor markers and mature markers was for the most part mutually exclusive.

**Table 2. Results depicting differentiation efficiency (44 days in differentiation culture).**

| **Marker** | **Cell type** | **Expression at the end-point of the study** |
|---|---|---|
| p63 | Corneal epithelial precursor cells | 70% (stDev 4%) |
| CK15 | | 60% (stDev 11%) |
| CK3 | Mature corneal epithelial cells | 35% (stDev 25%) |
| CK12 | | 70% (stDev 5%) |

From these results, it can be concluded, that the present method for culturing corneal epithelial precursor cells or mature corneal epithelial cells, comprising culturing stem cells in a culture medium comprising a TGF-beta inhibitor, a Wnt-inhibitor and a fibroblast growth factor as supplements has shown interesting potential.

According to one embodiment said method is characterized by expression of corneal epithelial marker p63, preferably with at least 65% and more preferably at least 70% of cells of the population positive with said marker. According to another embodiment said method is characterized by expression of corneal epithelial marker CK15, preferably with at least 50% and more preferably at least 70% of cells of the population positive with said marker. According to a further embodiment, said method is characterized by simultaneous expression of corneal epithelial marker p63, preferably with at least 65% and more preferably at least 70%, and corneal epithelial marker CK15, preferably with at least 50% and more preferably at least 70 % of cells of the population positive with said marker.

According to another embodiment said method is characterized by expression of corneal epithelial marker CK3, preferably with at least 50 % and more preferably at least 60% of cells of the population positive with said marker. According to yet another embodiment said method is characterized by expression of corneal epithelial marker CK12, preferably with at least 65 % and more preferably at least 75% of cells of the population positive with said marker. According to a further embodiment, said method is characterized by simultaneous expression of corneal epithelial marker CK3, preferably with at least 50% and more preferably at least 60%, and corneal epithelial marker CK12, preferably with at least 65% and more preferably at least 75% of cells of the population positive with said marker.

However, these results representing the end point of the present study, together with results describing p63-expression throughout the course of the study, indicate that it is within competence of a man skilled in the art to determine the time point providing desired enriched population, whether eye precursor cells, corneal epithelium precursors or corneal epithelial cells.

Hence, the present culture medium may be used for producing eye precursor cells or population thereof, corneal epithelial precursor cells or population thereof, corneal epithelial like cells or population thereof, or stratified corneal epithelium from pluripotent stem cells.
It will be obvious to a person skilled in the art that, as the technology advances, the inventive concept can be implemented in various ways. The invention and its embodiments are not limited to the examples described above but may vary within the scope of the claims.

### References

Ahmad S, Stewart R, Yung S, Kolli S, Armstrong L, Stojkovic M, Figueiredo F & Lako M (2007): Differentiation of human embryonic stem cells into corneal epithelial-like cells by in vitro replication of the corneal epithelium stem cell niche. Stem Cells 25: 1145-1155.
Bilic J, Izpisua Belmonte JC (2012), Induced pluripotent stem cells versus embryonic stem cells: close enough or yet too far apart? Stem Cells. 2012 Jan;30(1):33-41. doi: 10.1002/stem.700.
Hanson C, Hardarson T, Ellerstrom C, Nordberg M, Caisander G, Rao M, Hyllner J and Stenevi U (2012), Transplantation of human embryonic stem cells onto a partially wounded human cornea in vitro. Acta Ophthalmol 1-4.
Hayashi R, Ishikawa Y, Ito M, Kageyama T, Takashiba K, et al. (2012) Generation of Corneal Epithelial Cells from Induced Pluripotent Stem Cells Derived from Human Dermal Fibroblast and Corneal Limbal Epithelium. PLoS ONE 7(9): e45435. doi:10.1371/journal.pone.0045435.
Maminishkis A, Chen S, Jalickee S, Banzon T, Shi G, Wang FE, Ehalt T, Hammer JA, and Miller SS (2006), Confluent Monolayers of Cultured Human Fetal Retinal Pigment Epithelium Exhibit Morphology and Physiology of Native Tissue. Invest Ophthalmol Vis Sci. 47(8): 3612-3624.
Osaka F, Ikeda H, Mandai M, Wataya T, Watanabe K, Yoshimura N, Akaike A, Sasai Y, Takahashi M. Toward the Generation of Rod and Cone Photoreceptors from Mouse, Monkey and Human Embryonic Stem Cells. Nat Biotechnol. 2008 Feb;26(2):215-24.
Osakada F, Jin ZB, Hirami Y, Ikeda H, Danjyo T, Watanabe K, Sasai Y, Takanashi M. in vitro Differentiation of Retinal cells from Human Pluripotent Stem Cells by Small-Molecule Induction. J Cell Sci. 2009 Sep 1;122(pt 17):3169-79.
Rajala, K., Lindroos, B., Hussein, S. Lappalainen, RS., Pekkanen-Mattila, M., Inzunza, H., Miettinen, M., Narkilahti, S., Kerkelä, E., Aalto-Setälä, K., Otonkoski, O., Suuronen, R., Hovatta O., Skottman, H. A Defined and Xeno-free Culture Method Enabling the Establishment of Clinical-grade Human Embryonic, Induced Pluripotent and Adipose Derived Stem Cells. PLoS One. 2010.
Skottman, H. Derivation and characterization of three new human embryonic stem cell lines in Finland. In Vitro Cell Dev Biol Anim. 2010 Apr;46(3-4):206-9. Epub 2010 Feb 23.
Vaajasaari H, Ilmarinen T, Juuti-Uusitalo K, Rajala K, Onnela N, Narkilahti S, Suur-onen R, Hyttinen J, Uusitalo H, Skottman H. Toward the defined and xeno-free differ-entiation of functional human pluripotent stem cell-derived retinal pigment epithelial cells. Mol Vis. 2011 Feb 22;17:558-75.
Yoshida S, Yasuda M, Miyashita H, Ogawa Y, Yoshida T, et al. (2011) Generation of Stratified Squamous Epithelial Progenitor Cells from Mouse Induced Pluripotent Stem Cells. PLoS ONE 6(12): e28856. doi:10.1371/journal.pone.0028856.

## Claims

1. A method of producing differentiated eye cells selected from the group consisting of corneal epithelial precursor cells, corneal epithelial cells and stratified corneal epithelium the method comprising:
a) culturing pluripotent stem cells in an induction medium comprising a TGF-beta inhibitor, a Wnt inhibitor and a fibroblast growth factor thereby producing eye precursor cells;
b) culturing the eye precursor cells obtained in step a) in a cell culture medium comprising one or more supplements selected from the group consisting of epidermal growth factor (EGF), hydrocortisone, insulin, isoproterenol, and tri-iodo-thyronine, wherein the medium does not contain any of the following supplements: a TGF-beta inhibitor, a Wnt inhibitor and a fibroblast growth factor, thereby producing corneal epithelial precursor cells, wherein said corneal epithelial precursor cells express corneal epithelial marker p63, said expression being preferably quantified with immunofluorescent staining, and optionally maturating said corneal epithelial precursor cells into mature corneal epithelial cells or into corneal stratified epithelium.

2. The method according to claim 1, wherein the TGF-beta inhibitor is selected from TGF-beta inhibitors having molar mass of less than 800 g/mol, preferably less than 500 g/mol.

3. The method according to claim 2, wherein the TGF-beta inhibitor is selected from organic molecules according to Formula I 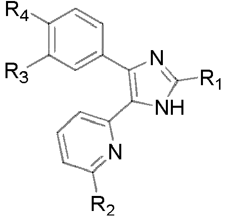 wherein R₁ represents an C₁-C₅ aliphatic alkyl group, carboxylic acid, amide, and R₂ represents an C₁-C₅ aliphatic alkyl, R₃ and R₄ represent aliphatic alkyls including heteroatoms, O or N, which may be linked together to form a 5- or 6-member heteroring.

4. The method according to any one of claims 1 to 3, wherein the Wnt inhibitor is selected from Wnt inhibitors having molar mass of less than 800 g/mol, preferably less than 500 g/mol.

5. The method according claim 4, wherein the Wnt inhibitor is selected from organic molecules according to Formula III 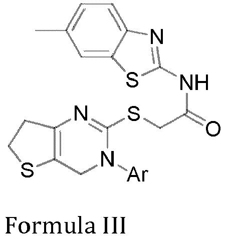 wherein Ar refers to substituted or unsubstituted aryl group.

6. The method according to any one of the preceding claims, wherein fibroblast growth factor is selected from basic FGF and synthetic small peptides exhibiting fibroblast growth factor-like activity.

7. The method according to any one of the preceding claims, wherein the content of the TGF-beta inhibitor is from 1 µM to 100 µM, preferably from 1 to 30 µM, the Wnt- inhibitor is from 1 µM to 100 µM, preferably from 1 µM to 30 µM, and the content of fibroblast growth factor is from 1 ng/ml to about 1000 ng/ml, preferably about 2 ng/ml to about 100 ng/ml , and more preferably about 30 ng/ml to about 80 ng/ml.

8. The method according to any one of the preceding claims, wherein the stem cells are selected from induced pluripotent stem (iPS) cells and embryonic stem (ES) cells, with the proviso that if human embryonic stem (hES) cells are used, the method does not include the destruction of human embryos.

9. The method according any one of the preceding claims, wherein the pluripotent stem cells are cultured for about four to about seven days.

10. The method according to claim 1, wherein said culturing in step b) is carried out on a substrate coated with an extra cellular matrix (ECM) protein selected from collagen IV, collagen I, laminin, vitronectin, fibronectin, and Matrigel™.

11. The method according to claim 1, wherein the corneal epithelial precursor cells expressing said marker p63 represent at least 65%, preferably at least 75%, most preferably at least 90% of the total cell population obtained.

12. The method according to any one of claims 1 to 11, which is performed in substantially xeno-free, substantially serum-free and/or defined conditions.

## Patentansprüche

1. Ein Verfahren zur Herstellung von differenzierten Augenzellen ausgewählt aus der Gruppe bestehend aus Hornhaut-Epithelvorläuferzellen, Hornhaut-Epithelzellen und geschichtetem Hornhaut-Epithel, das Verfahren umfassend:
a) Kultivieren von pluripotenten Stammzellen in einem Induktionsmedium, das einen TGF-Beta-Inhibitor, einem Wnt-Inhibitor und einen Fibroblasten-Wachstumsfaktor umfasst, wodurch Augenvorläuferzellen hergestellt werden;
b) Kultivieren der in Schritt a) erhaltenen Augenvorläuferzellen in einem Zeilkulturmedium, das ein oder mehrere Ergänzungen ausgewählt aus der Gruppe bestehend aus einem epidermalen Wachstumsfaktor (EGF), Hydrocortison, Insulin, Isoproterenol und Triiodothyronin umfasst, wobei das Medium keines der folgenden Ergänzungen enthält: einen TGF-Beta-Inhibitor, einen Wnt-Inhibitor und einen Fibroblasten-Wachstumsfaktor, wodurch Hornhaut-Epithelvorläuferzellen hergestellt werden, wobei die besagten Hornhaut-Epithelvorläuferzellen einen Hornhaut-Epithelmarker p63 exprimieren, wobei die besagte Expression bevorzugt mit Immunfluoreszenzfärbung quantifiziert wird, und optional Reifen der besagten Hornhaut-Epithelvorläuferzellen in Hornhaut-Epithelzellen oder in ein geschichtetes Hornhaut-Epithel.

2. Das Verfahren nach Anspruch 1, wobei der TGF-Beta-Inhibitor aus TGF-Beta-Inhibitoren mit einer molaren Masse von weniger als 800 g/Mol, bevorzugt weniger als 500 g/Mol ausgewählt wird.

3. Das Verfahren nach Anspruch 2, wobei der TGF-Inhibitor ausgewählt wird aus organischen Molekülen nach Formel 1 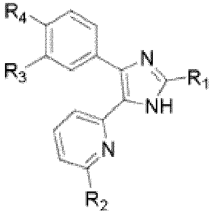 wobei R₁ eine aliphatische C₁-C₅-Alkyl-Gruppe, Karbonsäure, Amide darstellt und R₂ ein aliphatisches C₁-C₅-Alkyl darstellt und R₃ und R₄ aliphatische Alkyle umfassend Heteroatome 0 oder N darstellen, die miteinander verbunden sein können, um einen 5- oder 6-Element-Heteroring zu bilden.

4. Das Verfahren nach einem der Ansprüche 1 bis 3, wobei der Wnt-Inhibitor aus Wnt-Inhibitoren mit einer molaren Masse von weniger als 800 g/Mol, bevorzugt weniger als 500 g/Mol ausgewählt wird.

5. Das Verfahren nach Anspruch 4, wobei der Wnt-Inhibitor ausgewählt wird aus organischen Molekülen nach Formel III 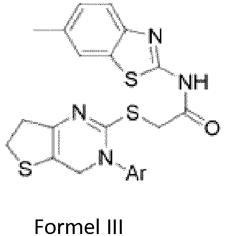 wobei sich Ar auf eine substituierte oder nicht substituierte Aryl-Gruppe bezieht.

6. Das Verfahren nach einem der vorangegangenen Ansprüche, wobei der Fibroblasten-Wachstumsfaktor aus basischem FGF und kleinen synthetischen Peptiden ausgewählt wird, die eine Fibroblasten-Wachstumsfaktor-ähnliche Aktivität zeigen.

7. Das Verfahren nach einem der vorangegangenen Ansprüche, wobei der Gehalt von dem TGF-Beta-Inhibitor von 1 µM bis 100 µM, bevorzugt von 1 bis 30 µM ist, der Wnt-Inhibitor von 1 µM bis 100 µM, bevorzugt von 1 µM bis 30 µM, ist und der Gehalt von dem Fibroblasten-Wachstumsfaktor von 1 ng/ml bis etwa 1000 ng/ml, bevorzugt etwa 2 ng/ml bis etwa 100 ng/ml und noch bevorzugter etwa 30 ng/ml bis etwa 80 ng/ml ist.

8. Das Verfahren nach einem der vorangegangenen Ansprüche, wobei die Stammzellen aus induzierten pluripotenten Stamm-(iPS)-Zellen und embryonalen Stamm-(ES)-Zellen ausgewählt werden, mit der Bedingung, dass, wenn menschliche embryonale Stamm-(hES)-Zellen verwendet werden, das Verfahren Verfahren nicht die Zerstörung von menschlichen Embryos umfasst.

9. Das Verfahren nach einem der vorangegangenen Ansprüche, wobei die pluripotenten Stammzellen für etwa vier bis etwa sieben Tage kultiviert werden.

10. Das Verfahren nach Anspruch 1, wobei das besagte Kultivieren in Schritt b) auf einem Substrat ausgeführt wird, das mit einem extrazellulären Matrix-(ECM)-Protein beschichtet ist, das ausgewählt wird aus Kollagen IV, Kollagen I, Laminin, Vitronektin, Fibronektion und Matrigel™.

11. Das Verfahren nach Anspruch 1, wobei die den besagten Marker p63 exprimierenden Hornhaut-Epithelvorläuferzellen wenigstens 65 %, bevorzugt wenigstens 75 %, noch bevorzugter wenigstens 90 % von der gesamten erhaltenen Zellpopulation darstellen.

12. Das Verfahren nach einem der Ansprüche 1 bis 11, welches unter im Wesentlichen Xeno-freien, im Wesentlichen Serum-freien und/oder definierten Bedingungen durchgeführt wird.

## Revendications

1. Procédé de production de cellules oculaires différenciées choisies dans le groupe constitué des cellules précurseurs épithéliales cornéennes, des cellules épithéliales cornéennes et de l'épithélium cornéen stratifié, le procédé comprenant :
a) la culture de cellules souches pluripotentes dans un milieu d'induction comprenant un inhibiteur de TGF-bêta, un inhibiteur de Wnt et un facteur de croissance de fibroblastes, produisant de la sorte des cellules précurseurs oculaires ;
b) la culture des cellules précurseurs oculaires obtenues à l'étape a) dans un milieu de culture cellulaire comprenant un ou plusieurs suppléments choisis dans le groupe constitué d'un facteur de croissance épidermique (EGF), de l'hydrocortisone, de l'insuline, de l'isoprotérénol et de la triiodothyronine, dans lequel le milieu ne contient pas l'un quelconque des suppléments suivants : un inhibiteur de TGF-bêta, un inhibiteur de Wnt et un facteur de croissance de fibroblastes, produisant de la sorte des cellules précurseurs épithéliales cornéennes, dans lequel lesdites cellules précurseurs épithéliales cornéennes expriment un marqueur épithélial cornéen p63, ladite expression étant de préférence quantifiée par coloration immunofluorescente, et
la maturation éventuelle desdites cellules précurseurs épithéliales cornéennes en cellules épithéliales cornéennes matures ou en épithélium cornéen stratifié.

2. Procédé selon la revendication 1, dans lequel l'inhibiteur de TGF-bêta est choisi parmi les inhibiteurs de TGF-bêta ayant une masse molaire inférieure à 800 g/mole, de préférence inférieure à 500 g/mole.

3. Procédé selon la revendication 2, dans lequel l'inhibiteur de TGF-bêta est choisi parmi des molécules organiques selon la formule (I) : 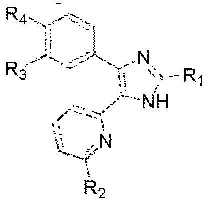 où R₁ représente un groupement alkyle aliphatique en C₁-C₅, un acide carboxylique, un amide et R₂ représente un alkyle aliphatique en C₁-C₅, R₃ et R₄ représentent des alkyles aliphatiques comprenant des hétéroatomes O ou N qui peuvent être liés à l'un à l'autre pour former un hétérocycle à 5 ou 6 chaînons.

4. Procédé selon l'une quelconque des revendications 1 à 3, dans lequel l'inhibiteur de Wnt est choisi parmi les inhibiteurs de Wnt ayant une masse molaire inférieure à 800 g/mole, de préférence inférieure à 500 g/mole.

5. Procédé selon la revendication 4, dans lequel l'inhibiteur de Wnt est choisi parmi des molécules organiques selon la formule (III) : 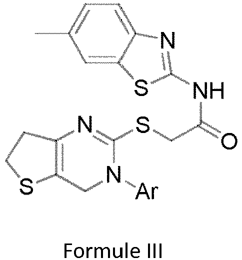 où Ar se réfère à un groupement aryle substitué ou non substitué.

6. Procédé selon l'une quelconque des revendications précédentes, dans lequel le facteur de croissance de fibroblastes est choisi parmi un FGF basique et des petits peptides synthétiques présentant une activité de type facteur de croissance de fibroblastes.

7. Procédé selon l'une quelconque des revendications précédentes, dans lequel la teneur en inhibiteur de TGF-bêta est de 1 µM à 100 µM, de préférence de 1 à 30 µM, la teneur en inhibiteur de Wnt est de 1 µM à 100 µM, de préférence de 1 µM à 30 µM et la teneur en facteur de croissance de fibroblastes est de 1 ng/ml à environ 1000 ng/ml, de préférence d'environ 2 ng/ml à environ 100 ng/ml, mieux encore d'environ 30 ng/ml à environ 80 ng/ml.

8. Procédé selon l'une quelconque des revendications précédentes, dans lequel les cellules souches sont choisies parmi les cellules souches pluripotentes induites (iPS) et les cellules souches embryonnaires (ES), à condition que, si l'on utilise des cellules souches embryonnaires humaines (hES), le procédé ne comprenne pas la destruction d'embryons humains.

9. Procédé selon l'une quelconque des revendications précédentes, dans lequel les cellules souches pluripotentes sont cultivées pendant environ quatre à environ sept jours.

10. Procédé selon la revendication 1, dans lequel ladite culture à l'étape b) est effectuée sur un substrat revêtu d'une protéine de matrice extracellulaire (ECM) choisie parmi le collagène IV, le collagène I, la laminine, la vitronectine, la fibronectine et le Matrigel™.

11. Procédé selon la revendication 1, dans lequel les cellules précurseurs épithéliales cornéennes exprimant ledit marqueur p63 représentent au moins 65 %, de préférence au moins 75 %, mieux encore au moins 90 % de la population totale de cellules obtenue.

12. Procédé selon l'une quelconque des revendications 1 à 11, qui est effectuée dans des conditions sensiblement non xénogéniques, sensiblement sans sérum et/ou définies.
